# EUROPEAN PATENT SPECIFICATION

(11) **EP 4 121 154 B1**
(45) Date of publication and mention of the grant of the patent: **17.07.2024**
(21) Application number: 21715074.7
(22) Date of filing: 08.03.2021
(51) Int. Cl.: A61M 16/10, A61M 16/00, A61M 16/20, A61M 16/06, A61M 16/12

(54) **SYSTEM FOR PROVIDING CONTROL OF SCAVENGING OF WASTE ANESTHETIC GAS IN A GAS MACHINE FOR ANESTHESIA OR ANALGESIA**
SYSTEM ZUR KONTROLLE DER AUFFÜLLUNG VON ANÄSTHETISCHEM ABFALLGAS IN EINER GAMASCHINE FÜR ANÄSTHESIE ODER ANALGESIE
SYSTÈME PERMETTANT DE CONTROLER LE LEVAGE DES DECHETS DE GAZ ANESTHESIQUE DANS UNE MACHINE A GAZ POUR L'ANESTHÉSIE OU L'ANALGESIE

(30) Priority: 17.03.2020 US 202062990620 P
(43) Date of publication of application: 25.01.2023
(73) Proprietor: Parker-Hannifin Corporation, Cleveland, Ohio 44124 (US)
(72) Inventor: SANGARE, Abdoulaye, Thorndale, Pennsylvania 19372 (US); WOLF, Seth B., North Wales, Pennsylvania 19454 (US); KORNOCK, Keith, Sr., Warrington, Pennsylvania 18976 (US)
(74) Representative: Murgitroyd & Company
(86) International application number: PCT/US2021/021311
(87) International publication number: WO 2021/188313

(56) References cited:
- WO-A1-99/62403
- US-A1- 2006 093 785
- US-A1- 2015 075 525

## Description

### Field of Invention

The present application generally relates to gas machines used to deliver gaseous anesthesia or analgesia, such as for example nitrous oxide, in medical and dental procedures, and more particularly to devices for scavenging waste anesthetic gas associated with the use of such gas machines.

### Background of the Invention

The present application relates to devices used for delivering gaseous anesthesia or analgesia in medical and dental applications, such as for example nitrous oxide delivery systems. More particularly, the present application relates to the scavenging of waste anesthetic gas that typically takes place during administration of the anesthetic gas to limit chronic exposure of the anesthetic gas to healthcare providers. The use of a gas machine to administer nitrous oxide or other gaseous anesthesia to patients requires an effective scavenging system to remove waste anesthetic gas from operatories. Chronic exposure to nitrous oxide has been associated with detrimental long-term reproductive health effects in healthcare providers. The National Institute for Occupational Safety and Health (NIOSH) recommends an exposure limit (REL) for nitrous oxide at 25 ppm as a time-weighted average (TWA) during the period of administration, and similar regulations are applicable in other countries. Exhaust ventilation of 40-45 L/min of air flow from the patient's mask is recommended to control occupational exposure to waste anesthetic gas that is not breathed in or is exhaled by the patient.

Conventional scavenging control systems include a vacuum control block with connections to both the exhalation line of a breathing circuit apparatus and a vacuum source. The user interface typically consists of a rotary knob and a pressure indicator. The rotary knob is provided to adjust the size of an orifice that exposes the exhalation line of the breathing circuit to the vacuum source. By increasing or decreasing the size of the orifice, the user is able to adjust the amount of vacuum suction, and thus implementing the air flow through the exhalation line of the breathing circuit. The pressure indicator, which typically is configured as a pressure gauge or ball float, is provided with a manufacturer-recommended range, within which the vacuum through the exhalation line of the breathing circuit needs to be adjusted. The recommended range has been determined to correspond to an air flow rate of 40-45 L/min based on testing conducted by the manufacturer under controlled conditions. In addition, some conventional scavenging control systems automatically provide vacuum to the breathing circuit as soon as gas is delivered to the patient. However, this feature is limited to an automatic on/off function for vacuum flow when the patient is breathing, but does not otherwise provide for accurate flow control and feedback of the flow rate to the operator.

The following documents may provide technical background to the present disclosure: US 2006/093785 WO 99/062403; and US 2015/075525.

Accordingly, in the various conventional systems an actual air flow rate is not measured and conveyed to the operator during administration of the anesthetic gas, and thus the operator is unable to ascertain whether an air flow rate of 40-45 L/min actually is achieved. Furthermore, different breathing circuits or masks may result in different air flow rates under the same vacuum conditions, which cannot be accounted for in conventional scavenging systems. Accordingly, conventional scavenging systems do not provide an accurate indication and control of scavenging of the waste anesthetic gas.

### Summary of the Invention

Accordingly, there is a need in the art for an improved scavenging system in devices used for delivering anesthetic gas in medical and dental applications. More particularly, the present application relates to the scavenging of waste anesthetic gas that typically takes place during administration of the anesthetic gas to limit chronic exposure of the gas to healthcare providers, in a manner that provides more accurate monitoring and control of the scavenging of waste anesthetic gas, including providing accurate feedback to the user regarding actual flow rate of the waste gas ventilation.

As used herein, the phrases "scavenging control system" and "scavenger" are employed synonymously.

The invention is defined by the appended claims. Subject-matter referred as embodiments and/or disclosures which are not claimed are not part of the invention.

Aspects of the invention include an anesthetic gas delivery system as defined in appended claim 1. The anesthetic gas delivery system includes a gas machine for supplying anesthetic gas to a patient; a scavenging control system that controls a level of vacuum suction to evacuate waste anesthetic gas that is not breathed in by the patient; and a user interface electronically coupled to the scavenging control system. The scavenging control system is configured to adjust the level of vacuum suction to adjust the flow rate of the waste anesthetic gas through the scavenging control system based on a measured flow rate of the waste anesthetic gas that is measured externally or remotely from the gas machine. The scavenging control system further is configured to transmit flow rate information corresponding to the measured flow rate to the user interface, and the user interface is configured to output the flow rate information to convey the flow rate information to an operator of the anesthetic gas delivery system.

The scavenging control system includes an air flow sensor remote or external from the gas machine that measures the flow rate of the waste anesthetic gas through the scavenging control system; a control valve, such as a proportional solenoid valve, that is controllable to adjust the level of vacuum suction to adjust the flow rate of the waste anesthetic gas; and electronic control circuitry that is configured to receive a measured flow rate from the sensor and to control the control valve to adjust the level of vacuum suction based on the flow rate measured by the sensor. The electronic control circuitry further is configured to transmit the flow rate information corresponding to the flow rate measured by the sensor to the user interface. The electronic control of the control valve by the electronic control circuitry may be implemented in one or both of a closed-loop or open-loop control implementation based on the actual flow rate of the waste anesthetic gas as measured by the sensor.

These and further features of the present invention will be apparent with reference to the following description and attached drawings. In the description and drawings, particular embodiments of the invention have been disclosed in detail as being indicative of some of the ways in which the principles of the invention may be employed, but it is understood that the invention is defined by the appended claim.

Features that are described and/or illustrated with respect to one embodiment may be used in the same way or in a similar way in one or more other embodiments and/or in combination with or instead of the features of the other embodiments.

### Brief Description of the Drawings

Fig. 1 is a drawing depicting a perspective view of an exemplary anesthetic gas delivery system in accordance with embodiments of the present application.
Fig. 2 is a drawing depicting a perspective view of an alternative configuration of an anesthetic gas delivery system in accordance with embodiments of the present application.
Fig. 3 is a drawing depicting a first perspective view of a scavenging control system that may be used in the delivery systems of Figs. 1 and 2.
Fig. 4 is a drawing depicting a second perspective view of the scavenging control system of Fig. 3.
Fig. 5 is a drawing depicting a side cross-sectional view of the scavenging control system of Figs. 3 and 4.

### Detailed Description

Embodiments of the present application will now be described with reference to the drawings, wherein like reference numerals are used to refer to like elements throughout. It will be understood that the figures are not necessarily to scale.

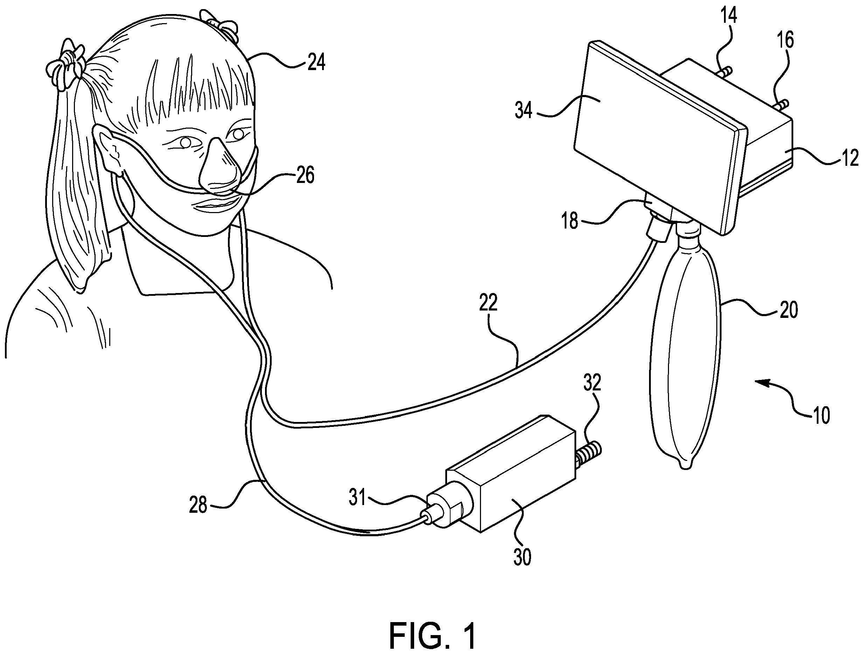

The present application provides an improved scavenging system for devices used for delivering anesthetic gas in medical and dental applications to limit chronic exposure of the waste anesthetic gas to healthcare providers. The scavenging system provides more accurate monitoring and control of the scavenging of waste anesthetic gas, including providing accurate feedback to the operator regarding actual flow of the waste gas ventilation. Fig. 1 is a drawing depicting a perspective view of an exemplary anesthetic gas delivery system 10 in accordance with embodiments of the present application. The anesthetic gas delivery system 10 includes a continuous-flow gas machine 12 that provides an anesthetic gas, such as for example nitrous oxide gas mixed with oxygen gas. In an example of a nitrous oxide delivery system, the gas machine 12 includes a first gas inlet 14 that receives a flow of nitrous oxide, and a second gas inlet 16 that receives a flow of oxygen. The gas machine 12 operates to mix the nitrous oxide and oxygen together within a chamber defined by of the gas machine to form a mixed anesthetic gas that is suitable for a patient as is known in the art.

The delivery system 10 further includes a bag tee 18 that is in fluid communication with the gas machine 12. The bag tee 18 is essentially a "tee-shaped" connector which thus includes three connection portions. A first connection of the bag tee 18 provides the fluid communication to the gas machine 12 to receive the mixed anesthetic gas. A second connection of the bag tee 18 connects to a reservoir bag 20, whereby the mixed anesthetic gas flows into and fills up the reservoir bag 20 for delivery to the patient. A third connection of the bag tee 18 connects to a breathing circuit apparatus that provides the mixed anesthetic gas to a patient through a nasal mask, and evacuates waste anesthetic gas to a scavenger as further detailed below. Generally, the breathing circuit apparatus may include an inhalation line, a nasal mask worn by a patient, and an evacuation line. As illustrated in Fig. 1, for example, the third connection of the bag tee 18 is connected to an inhalation line 22 that supplies the anesthetic gas to a patient 24 through a nasal mask 26. The nasal mask 26 then is connected to an evacuation line 28 that is in fluid communication with a scavenging control system or scavenger 30 for the evacuation or ventilation of waste anesthetic gas.

Not all of the mixed anesthetic gas actually is inhaled and metabolized by the patient, and thus excess anesthetic gas, also referred to as the waste anesthetic gas, flows through the evacuation line 28 to the scavenging control system 30. The scavenging control system 30 has a line connector 31 that is connected to the evacuation line 28 from which the scavenging control system receives waste anesthetic gas. The scavenging control system 30 also has a vacuum connector 32 that is connectable to a vacuum source line (not shown), and the waste anesthetic gas is removed from the delivery system under the vacuum suction so as not to detrimentally expose the healthcare provider to the anesthetic gas.

The delivery system 10 further includes a user interface 34 that is mounted to the gas machine 12. The user interface 34 may include one or more of a display to display information about the operation of the delivery system, a touchscreen for additionally implementing control functions as part of the display functionality, a keypad for entering control commands, dedicated control inputs (buttons, pads, etc.), visual and/or audio indicators and alerts as to system performance, and the like as are common in medical device instruments.

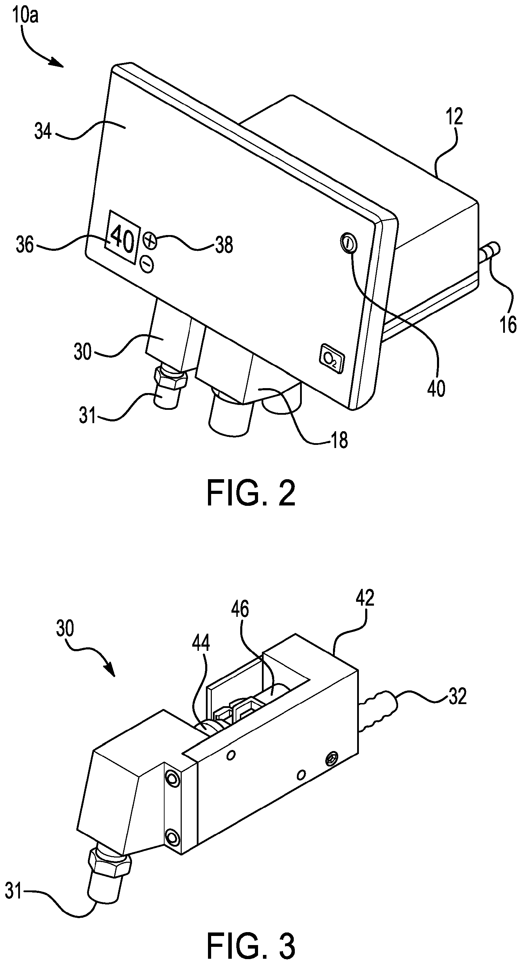

The precise assembly configuration of the anesthetic gas delivery system may be varied as is suitable for any particular implementation. For example, Fig. 2 is a drawing depicting a perspective view of an alternative configuration of an anesthetic gas delivery system 10a (for convenient illustration the breathing apparatus components 22/26/28 and the reservoir bag 20 are omitted from Fig. 2). In the configuration of the delivery system 10a, the scavenging control system 30 is mounted directly to the gas machine 12 and adjacent to the bag tee 18. Other variations may be employed. For example, the scavenging control system 30 and the bag tee 18 may be combined into a single unit and mounted directly to the gas machine 12 as an integral sub-assembly, or the scavenging control system 30 and/or the bag tee 18 may be mounted to an external mounting separate or remote from the gas machine 12, with an additional fluid line for fluid connection of the bag tee to the gas machine 12. Accordingly, any suitable variations of the assembly configuration of the anesthetic gas delivery system may be employed.

Fig. 2 further depicts an example implementation of the user interface 34. In this particular example, the user interface 34 includes a scavenger flow rate display 36, scavenger flow rate controls 38, and other system controls 40 (e.g., a power button is depicted as an example). As referenced above, the user interface, including the flow rate display and controls and any other system controls, may be implemented using one or more of a display, touchscreen, keypad, visual/audio indicators, and/or other dedicated informatory and control mechanisms as are common in medical device instruments.

As referenced above, NIOSH recommends an exposure limit (REL) for nitrous oxide at 25 ppm as a time-weighted average (TWA) during the period of administration, and exhaust ventilation of 40-45 L/min of air flow from the patient's mask is recommended to limit occupational exposure to waste anesthetic gas that is not breathed in or that is exhaled by the patient. As an example, therefore, the flow rate display 36 depicts "40" (i.e., 40 L/min) to demonstrate a flow rate within the recommended range. It will be appreciated that the flow rate may deviate from the recommended range depending upon particular circumstances and conditions of operation. Accordingly, the actual flow rate is displayed on the display 36 of the user interface 34 to convey to the operator whether the delivery system is functioning properly and within the recommended flow rate range. A flow rate that is too low may not adequately exhaust the waste anesthetic gas, which can lead to overexposure of the operator to the anesthetic gas. A flow rate that is too high may draw in too much of the anesthetic gas, which is inefficient.

This actual flow rate is determined in part by the strength of the vacuum suction that is applied through the scavenging control system 30. Accordingly, the scavenger flow rate controls 38 may be employed to adjust the vacuum strength to increase or decrease the vacuum suction, and thereby decrease or increase the flow rate, as may be warranted to maintain the flow rate within the recommended range. In the example depicted in Fig. 2, the flow rate controls 38 are implemented with "+" and "-" control features, which for example may be touchscreen icons or physical input buttons or pads, which constitute flow rate control commands to increase or decrease the flow rate. Any suitable configuration of flow rate controls 38 may be used to input flow rate control commands to adjust the flow rate. Conventional scavenging control systems lack such features, in that an actual air flow rate is not measured and conveyed to the operator during administration of the anesthetic gas, and thus the operator is unable to ascertain whether an air flow rate within the recommended range actually is achieved. Accordingly, conventional scavenging systems do not provide an accurate indication and control of scavenging of the waste anesthetic gas.

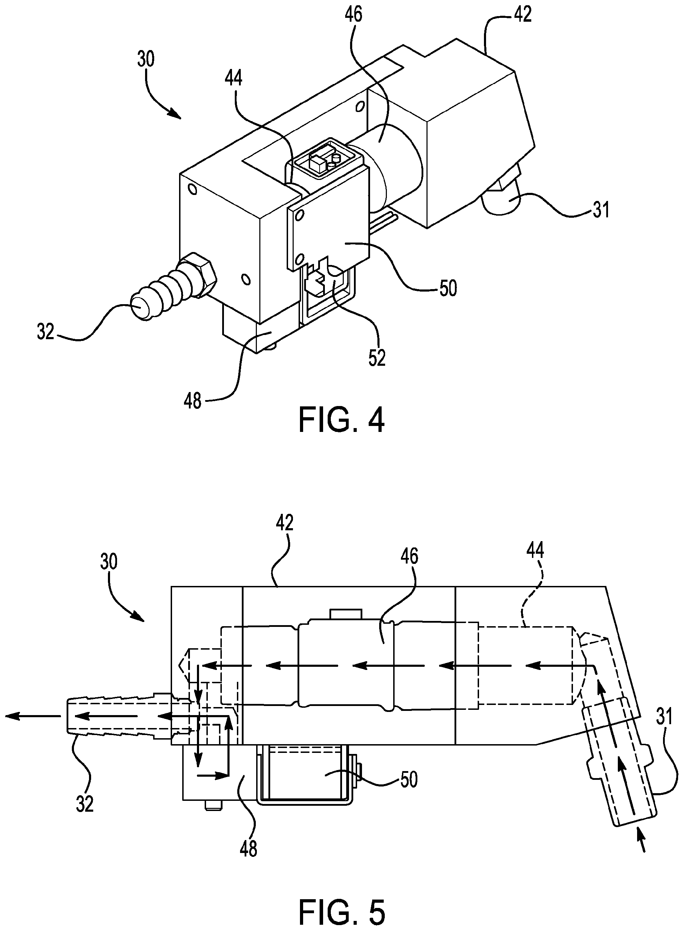

Fig. 3 is a drawing depicting a first perspective view of the scavenging control system 30 in isolation, which may be used in the delivery systems of Figs. 1 and 2. Fig. 4 is a drawing depicting a second perspective view of the scavenging control system 30, and Fig. 5 is a drawing depicting a side cross-sectional view of the scavenging control system 30. In the depicted example, the scavenging control system 30 includes a housing 42 that provides a support or mount for the other components of the scavenging control system. Accordingly, the line connector 31 and the vacuum connector 32 extend from the housing 42. The scavenging control system 30 further includes a fluid passage 44 through the housing 42 (see particularly Fig. 5), e.g., that is supported by or defined within the housing 42. The fluid passage 44 extends between the line connector 31 and the vacuum connector 32 to provide a flow pathway from the evacuation line of the breathing circuit apparatus to the vacuum suction line for the evacuation or ventilation of the waste anesthetic gas.

The fluid passage 44 includes an air flow sensor 46 that senses the air flow rate of the waste anesthetic gas through the scavenging control system 30. Certain conventional configurations have employed flow rate sensors provided within the gas machine to regulate the gas flow through the gas machine, whereas the air flow sensor 46 specifically is incorporated as part of the scavenging control system 30 to provide an enhanced measurement of the scavenging of the waste anesthetic gas. In other words, the air flow sensor 46 is encompassed within the housing 42 within the fluid passage 44 through the scavenger 30 to provide a more direct measurement of the scavenging flow, and thus the air flow sensor 46 is positioned external to or remote from the gas machine 12. The air flow sensor, for example, may be a thermal sensor.

The flow path of the waste anesthetic gas through the scavenger control system is indicated generally by the arrowed line in Fig. 5. Flow proceeds from the air flow sensor 46 through a proportional solenoid valve 48, and subsequently exiting the scavenger through the vacuum connector 32. The solenoid valve 48 is configured as a proportional solenoid valve that is adjustable to control the level or strength of the vacuum suction, thereby adjusting the flow rate of the waste anesthetic gas. The scavenging control system 30 further includes electronic control circuitry including a microcontroller unit that controls the operation of the solenoid valve 48. The electronic control circuitry with the microcontroller unit may be configured in any suitable manner typically employed for solenoid valve control electronics. For example as depicted in Fig. 4, the electronic control circuitry may be configured as a printed circuit assembly 50 having an electrical connector 52 that receives a cable (not shown) for electrical connection of scavenger 30 to the gas machine 12.

The scavenging control system 30 operates as follows to control the flow rate of waste anesthetic gas to maintain a flow rate in accordance with recommendations for limiting healthcare provider exposure. As referenced above, the solenoid valve 48 is an adjustable proportional solenoid valve used to control a level of vacuum that is applied via the vacuum connector 32. The air flow sensor 46 measures the flow rate within the scavenger 30 of gas that is pulled through the evacuation line 28 of the breathing circuit apparatus and into the scavenging control system 30 by the vacuum. The electronic control circuitry 50 with the microcontroller acquires flow rate measurements from the air flow sensor 46. Based on the measured flow rate, the electronic control circuitry 50 controls the proportional solenoid valve 48 to adjust the vacuum suction level to achieve the desired flow rate.

The scavenging control system 30 communicates with the user interface 34 and the gas machine 12 through an electronic interface. By such electronic interface, the electronic control circuitry 50 can output flow rate information to the user interface 34 and receive flow rate control commands from the user interface 34. For example, referring to Fig. 2 in combination with Figs. 3-5, the actual flow rate as measured by the air flow sensor 46 through the scavenger 30 may be displayed on the display 36, and flow rate control commands to adjust or set the flow rate may be inputted by an operator using the scavenger flow rate controls 38. Inputs to the scavenger flow rate controls 38 are converted to electronic signal control commands that are transmitted to the electronic control circuitry 50 for control of the solenoid valve 48.

The scavenging control system 30 may be configured in one or both of a closed-loop or open-loop control implementation. In a closed-loop control implementation, the operator inputs a flow rate control command via the controls 38 that sets an air flow rate setpoint (e.g., the operator sets a predetermined flow rate within the recommended range), and the electronic control circuitry of the scavenger control system automatically controls the solenoid valve based on the air flow sensor measurements to achieve the desired setpoint. An operator still may monitor the actual flow rate via the display 36 of the user interface 34 to ensure proper operation. In an open-loop control implementation, the operator directly adjusts the scavenger's solenoid valve using the scavenger flow rate controls 38, which generate flow rate control commands for the electronic control circuitry to operate the solenoid valve in accordance with the inputs. The electronic control circuity reads the air flow sensor information to position the solenoid valve in accordance with the command signals from the scavenger flow rate controls. Again, the operator may monitor the actual flow rate via the user interface display. Accordingly, in both closed-loop and open-loop implementations, the actual air flow rate is measured by the air flow sensor of the scavenger and conveyed to the user via the gas machine user interface. In this regard, the user interface 34 further may include any suitable visual and/or audio indicators that can act as alerts or alarms when the flow rate is outside of the recommended range, so that an operator may adjust the flow rate using the flow rate controls or otherwise investigate or troubleshoot deficient system operation.

As another control functionality, the solenoid valve 48 also automatically is actuated as soon as the operator initiates the gas machine to provide a gas flow to the patient. Initiation of the gas flow, including either or both of the nitrous oxide flow and the oxygen flow, may be sensed by a dedicated sensor in the gas machine electronics, and an initiation command then may be transmitted electronically to the electronic control circuitry 50 to actuate the solenoid valve 48. Initiation of the gas flow alternatively may be sensed by the air flow sensor 46 of the scavenging control system 30 to actuate the solenoid valve 48. Accordingly, when at least one of the nitrous oxide flow and the oxygen flow is sensed as present, the scavenging control system operates to open the solenoid valve. Such operation, particularly in maintaining scavenging while the nitrous oxide flow is off, has an advantage for safe shutoff at the end of a patient procedure. Specifically at the end of such procedure, when the nitrous oxide flow is shut off with the oxygen flow maintained, the system continues to scavenge away any excess nitrous oxide that still may be present in the delivery system even after the nitrous oxide flow is shut off. The full scavenging of excess nitrous oxide after turning off the nitrous oxide flow, whether sourced from the reservoir bag of the delivery system or constituting non-metabolized nitrous oxide being exhaled by the patient, can take up to about ten minutes or more.

The gas delivery system with enhanced scavenging control system has advantages over conventional configurations. The conventional user interface of a rotary knob and a pressure indicator does not measure and convey the actual air flow rate to the operator during administration of the anesthetic gas, and thus the operator is unable to ascertain whether an air flow rate within the recommended range actually is achieved. Accordingly, conventional scavenging systems do not provide an accurate indication and control of scavenging of the waste anesthetic gas. In contrast, the scavenging control system of the present application conveys actual flow rate information to the operator as to the flow rate specifically within the scavenger, thereby permitting precise operator adjustment to the flow rate using the open-loop and/or closed-loop control as described above. In addition, the use of a proportional solenoid valve under electronic control results in enhanced accuracy of the flow rate control. Accordingly, a more effective and reliable control of the removal of the waste anesthetic gas is achieved as compared to conventional configurations.

The invention is defined in independent claim 1. Preferred embodiments are matter of the appended dependent claims.

## Claims

1. An anesthetic gas delivery system (10) comprising:
a gas machine (12) for supplying anesthetic gas to a patient;
a scavenger control system (30) that controls a level of vacuum suction to evacuate waste anesthetic gas; and
a user interface (34) electronically coupled to the scavenger control system (30);
wherein:
the scavenger control system (30) is configured to adjust the level of vacuum suction to adjust an actual flow rate of the waste anesthetic gas through the scavenger control system (30) based on a measured actual flow rate of the waste anesthetic gas external from the gas machine (12);
the scavenger control system (30) further is configured to transmit actual flow rate information corresponding to the measured actual flow rate to the user interface (34); and
the user interface (34) is configured to output the actual flow rate information to convey the actual flow rate information to an operator of the anesthetic gas delivery system, wherein the user interface (34) includes a scavenger flow rate control (38) for receiving an input of a flow rate control command, and the scavenger control system (30) adjusts the actual flow rate of the waste anesthetic gas in accordance with the flow rate control command;
wherein the scavenger control system (30) comprises:
an air flow sensor (46) positioned external from the gas machine (12) that measures the actual flow rate of the waste anesthetic gas through the scavenger control system (30);
a control valve (48) that is controllable to adjust the level of vacuum suction to adjust the actual flow rate of the waste anesthetic gas; and
electronic control circuitry (50) that is configured to receive a measured actual flow rate from the sensor (46) and to control the control valve (48) to adjust the level of vacuum suction based on the actual flow rate measured by the sensor (46);
wherein the electronic control circuitry (50) further is configured to transmit the actual flow rate information corresponding to the actual flow rate measured by the sensor (46) to the user interface (34).

2. The anesthetic gas delivery system (10) of claim 1, wherein the flow rate control command is a flow rate setpoint, and the scavenger control system (30) operates in accordance with closed-loop control to automatically adjust the actual flow rate of the waste anesthetic gas to achieve the flow rate setpoint.

3. The anesthetic gas delivery system (10) of claim 2, wherein the flow rate control command is a flow rate adjustment input to increase or decrease the actual flow rate of the waste anesthetic gas, and the scavenger control system (30) operates in accordance with open-loop control to adjust the actual flow rate in accordance with the flow rate adjustment input.

4. The anesthetic gas delivery system (10) of any preceding claim,
wherein the control valve (48) is a proportional solenoid valve (48).

5. The anesthetic gas delivery system (10) of any preceding claim,
wherein the electronic control circuitry (50) controls the control valve (48) to adjust the actual flow rate of the waste anesthetic gas in accordance with the flow rate control command.

6. The anesthetic gas delivery system (10) of claim 5, wherein the flow rate control command is a flow rate setpoint, and the scavenger control system (30) operates in accordance with closed-loop control whereby the electronic control circuitry (50) automatically controls the control valve (48) to achieve the flow rate setpoint.

7. The anesthetic gas delivery system (10) of claim 5 or claim 6, wherein the flow rate control command is a flow rate adjustment input to increase or decrease the actual flow rate, and the scavenger control system (30) operates in accordance with open-loop control whereby the electronic control circuitry (50) controls the control valve (48) in accordance with the flow rate adjustment input.

8. The anesthetic gas delivery system (10) of any of claims 1-7, wherein the user interface (34) includes a display (36) for displaying the flow rate information.

9. The anesthetic gas delivery system (10) of any of claims 1-8, further comprising a breathing apparatus in fluid communication with the gas machine (12) and the scavenger control system (30);
wherein the breathing apparatus comprises an inhalation line (22) that transmits the anesthetic gas from the gas machine (12) to the patient, a nasal mask (26) that is worn by the patient to inhale at least a portion of the anesthetic gas, and an evacuation line (28) that transmits the waste anesthetic gas from the nasal mask (26) to the scavenger control system (30).

10. The anesthetic gas delivery system (10) of claim 9, further comprising a bag tee (18) that is in fluid communication with the gas machine (12) and the breathing apparatus (22, 26, 28), and a reservoir bag (20) that is fluid communication with the bag tee (18), wherein the anesthetic gas flows through the bag tee (18) to fill up the reservoir bag (20).

11. The anesthetic gas delivery system (10) of any of claims 1-10, wherein the anesthetic gas delivery system is a nitrous oxide delivery system, the gas machine (12) has a first inlet (14) for receiving a supply of nitrous oxide and a second inlet (16) for receiving a supply of oxygen, and the gas machine (12) mixes the nitrous oxide and the oxygen to form a mixed anesthetic gas that is supplied to the patient.

12. The anesthetic gas delivery system (10) of any preceding claim, wherein the air flow sensor (46) is a thermal sensor.

## Patentansprüche

1. Ein Zufuhrsystem (10) für Anästhesiegas, das Folgendes beinhaltet:
eine Gasmaschine (12) zum Liefern von Anästhesiegas an einen Patienten;
ein Steuerungssystem (30) für ein Abfangmittel, das einen Grad an Vakuumsaugung steuert, um Abfall-Anästhesiegas zu evakuieren; und
eine Benutzerschnittstelle (34), die mit dem Steuerungssystem (30) für ein Abfangmittel elektronisch gekoppelt ist;
wobei:
das Steuerungssystem (30) für ein Abfangmittel konfiguriert ist, um den Grad an Vakuumsaugung einzustellen, um einen tatsächlichen Durchfluss des Abfall-Anästhesiegases durch das Steuerungssystem (30) für ein Abfangmittel basierend auf einem gemessenen tatsächlichen Durchfluss des Abfall-Anästhesiegases außerhalb der Gasmaschine (12) anzupassen;
das Steuerungssystem (30) für ein Abfangmittel ferner konfiguriert ist, um Informationen über den tatsächlichen Durchfluss, der dem gemessenen tatsächlichen Durchfluss an die Benutzerschnittstelle (34) entspricht, zu übertragen; und
die Benutzerschnittstelle (34) konfiguriert ist, um Informationen über den tatsächlichen Durchfluss auszugeben, um Informationen über den tatsächlichen Durchfluss an einen Betreiber des Zufuhrsystems für Anästhesiegas zu übermitteln, wobei die Benutzerschnittstelle (34) eine Durchflusssteuerung (38) für das Abfangmittel umfasst, um einen Eingang eines Durchflusssteuerungsbefehls zu empfangen, und das Steuerungssystem (30) für ein Abfangmittel den tatsächlichen Durchfluss des Abfall-Anästhesiegases gemäß dem Durchflusssteuerungsbefehl anpasst;
wobei das Steuerungssystem (30) für ein Abfangmittel Folgendes beinhaltet:
einen Luftstromsensor (46), der außerhalb der Gasmaschine (12) positioniert ist, der den tatsächlichen Durchfluss des Abfall-Anästhesiegases durch das Steuerungssystem (30) für ein Abfangmittel misst;
ein Steuerventil (48), das gesteuert werden kann, um den Grad an Vakuumsaugung anzupassen, um den tatsächlichen Durchfluss des Abfall-Anästhesiegases anzupassen; und
eine elektronische Steuerschaltung (50), die konfiguriert ist, um einen gemessenen tatsächlichen Durchfluss von dem Sensor (46) zu empfangen und das Steuerventil (48) zu steuern, um den Grad an Vakuumsaugung basierend auf dem tatsächlichen Durchfluss, der durch den Sensor (46) gemessen wird, anzupassen;
wobei die elektronische Steuerschaltung (50) ferner konfiguriert ist, um die tatsächlichen Informationen über den Durchfluss, die dem tatsächlichen Durchfluss entsprechen, der durch den Sensor (46) zu der Benutzerschnittstelle (34) gemessen wird, zu übertragen.

2. Zufuhrsystem (10) für Anästhesiegas gemäß Anspruch 1, wobei der Durchflusssteuerungsbefehl ein Durchflusssollwert ist, und das Steuerungssystem (30) für ein Abfangmittel gemäß geschlossenem Regelkreis betrieben wird, um den tatsächlichen Durchfluss des Abfall-Anästhesiegases anzupassen, um den Durchflusssollwert zu erreichen.

3. Zufuhrsystem (10) für Anästhesiegas gemäß Anspruch 2, wobei der Durchflusssteuerungsbefehl ein Durchflusssanpassungseingang ist, um den tatsächlichen Durchfluss des Abfall-Anästhesiegases zu vergrößern oder zu verkleinern, und das Steuerungssystem (30) für ein Abfangmittel gemäß der offenen Regelsteuerung betrieben wird, um den tatsächlichen Durchfluss gemäß dem Durchflussanpassungseingang anzupassen.

4. Zufuhrsystem (10) für Anästhesiegas gemäß einem der vorhergehenden Ansprüche, wobei das Steuerventil (48) ein proportionales Solenoidventil (48) ist.

5. Zufuhrsystem (10) für Anästhesiegas gemäß einem der vorhergehenden Ansprüche, wobei die elektronische Steuerschaltung (50) das Steuerventil (48) steuert, um den tatsächlichen Durchfluss des Abfall-Anästhesiegases gemäß dem Durchflusssteuerungsbefehl anzupassen.

6. Zufuhrsystem (10) für Anästhesiegas gemäß Anspruch 5, wobei der Durchflusssteuerungsbefehl ein Durchflusssollwert ist, und das Steuerungssystem (30) für ein Abfangmittel gemäß geschlossenem Regelkreis betrieben wird, wodurch die elektronische Steuerschaltung (50) das Steuerventil (48) automatisch steuert, um den Durchflusssollwert zu erreichen.

7. Zufuhrsystem (10) für Anästhesiegas gemäß Anspruch 5 oder Anspruch 6, wobei der Durchflusssteuerungsbefehl ein Durchflussanpassungseingang ist, um den tatsächlichen Durchfluss zu vergrößern oder zu verkleinern, und das Steuerungssystem (30) für ein Abfangmittel gemäß der offenen Regelsteuerung betrieben wird, wodurch die elektronische Steuerschaltung (50) das Steuerventil (48) gemäß dem Durchflussanpassungseingang steuert.

8. Zufuhrsystem (10) für Anästhesiegas gemäß einem der Ansprüche 1-7, wobei die Benutzerschnittstelle (34) eine Anzeige (36) zum Anzeigen der Durchflussinformationen umfasst.

9. Zufuhrsystem (10) für Anästhesiegas gemäß einem der Ansprüche 1-8, das ferner ein Atemgerät in Fluidkommunikation mit der Gasmaschine (12) und dem Steuerungssystem (30) für ein Abfangmittel beinhaltet;
wobei das Atemgerät eine Inhalationsleitung (22), die das Anästhesiegas von der Gasmaschine (12) zu dem Patienten überträgt, eine Nasenmaske (26), die durch den Patienten getragen wird, um mindestens einen Teil des Anästhesiegases zu inhalieren, und eine Evakuierungsleitung (28) beinhaltet, die das Abfall-Anästhesiegas von der Nasenmaske (26) zu dem dem Steuerungssystem (30) für ein Abfangmittel überträgt.

10. Zufuhrsystem (10) für Anästhesiegas gemäß Anspruch 9, das ferner ein beutelförmiges T-Stück (18), das sich in Fluidkommunikation mit der Gasmaschine (12) und dem Atemgerät (22, 26, 28) befindet, und einen Reservoirbeutel (20) beinhaltet, der sich in Fluidkommunikation mit dem beutelförmigen T-Stück (18) befindet, wobei das Anästhesiegas durch das beutelförmige T-Stück (18) strömt, um den Reservoirbeutel (20) zu füllen.

11. Zufuhrsystem (10) für Anästhesiegas gemäß einem der Ansprüche 1-10, wobei das Zufuhrsystem für Anästhesiegas ein Zufuhrsystem für Stickstoffoxid ist, die Gasmaschine (12) einen ersten Einlass (14) zum Empfangen einer Lieferung von Stickstoffoxid und einen zweiten Einlass (16) zum Empfangen einer Lieferung von Sauerstoff aufweist und die Gasmaschine (12) das Stickstoffoxid und den Sauerstoff mischt, um ein Anästhesiemischgas zu bilden, das an den Patienten geliefert wird.

12. Zufuhrsystem (10) für Anästhesiegas gemäß einem der vorhergehenden Ansprüche, wobei der Luftstromsensor (46) ein Wärmesensor ist.

## Revendications

1. Un système de distribution de gaz anesthésique (10) comprenant :
une machine à gaz (12) destinée à apporter du gaz anesthésique à un patient ;
un système de contrôle de récupérateur (30) qui contrôle un niveau d'aspiration sous vide pour évacuer du gaz anesthésique résiduel ; et
une interface utilisateur (34) couplée électroniquement au système de contrôle de récupérateur (30) ;
dans lequel :
le système de contrôle de récupérateur (30) est configuré pour ajuster le niveau d'aspiration sous vide afin d'ajuster un débit d'écoulement réel du gaz anesthésique résiduel à travers le système de contrôle de récupérateur (30) sur la base d'un débit d'écoulement réel mesuré du gaz anesthésique résiduel externe à la machine à gaz (12) ;
le système de contrôle de récupérateur (30) est en outre configuré pour transmettre des informations de débit d'écoulement réel correspondant au débit d'écoulement réel mesuré à l'interface utilisateur (34) ; et
l'interface utilisateur (34) est configurée pour émettre les informations de débit d'écoulement réel afin d'acheminer les informations de débit d'écoulement réel à un opérateur du système de distribution de gaz anesthésique, l'interface utilisateur (34) incluant un contrôle (38) de débit d'écoulement de récupérateur pour recevoir une entrée d'une commande de contrôle de débit d'écoulement, et le système de contrôle de récupérateur (30) ajustant le débit d'écoulement réel du gaz anesthésique résiduel conformément à la commande de contrôle de débit d'écoulement ;
dans lequel le système de contrôle de récupérateur (30) comprend :
un capteur d'écoulement d'air (46) positionné de façon externe à la machine à gaz (12) qui mesure le débit d'écoulement réel du gaz anesthésique résiduel à travers le système de commande de récupérateur (30) ;
une valve de contrôle (48) qui peut être contrôlée pour ajuster le niveau d'aspiration sous vide afin d'ajuster le débit d'écoulement réel du gaz anesthésique résiduel ; et
un circuit de contrôle électronique (50) qui est configuré pour recevoir un débit d'écoulement réel mesuré en provenance du capteur (46) et pour contrôler la valve de contrôle (48) afin d'ajuster le niveau d'aspiration sous vide sur la base du débit d'écoulement réel mesuré par le capteur (46) ;
dans lequel le circuit de contrôle électronique (50) est en outre configuré pour transmettre les informations de débit d'écoulement réel correspondant au débit d'écoulement réel mesuré par le capteur (46) à l'interface utilisateur (34).

2. Le système de distribution de gaz anesthésique (10) de la revendication 1, dans lequel la commande de contrôle de débit d'écoulement est un point de consigne de débit d'écoulement, et le système de contrôle de récupérateur (30) fonctionne conformément à un contrôle en boucle fermée pour ajuster automatiquement le débit d'écoulement réel du gaz anesthésique résiduel afin d'obtenir le point de consigne de débit d'écoulement.

3. Le système de distribution de gaz anesthésique (10) de la revendication 2, dans lequel la commande de contrôle de débit d'écoulement est une entrée d'ajustement de débit d'écoulement pour augmenter ou diminuer le débit d'écoulement réel du gaz anesthésique résiduel, et le système de contrôle de récupérateur (30) fonctionne conformément à un contrôle en boucle ouverte pour ajuster le débit d'écoulement réel conformément à l'entrée d'ajustement de débit d'écoulement.

4. Le système de distribution de gaz anesthésique (10) de n'importe quelle revendication précédente, dans lequel la valve de contrôle (48) est une valve à solénoïde proportionnelle (48).

5. Le système de distribution de gaz anesthésique (10) de n'importe quelle revendication précédente, dans lequel le circuit de contrôle électronique (50) contrôle la valve de contrôle (48) pour ajuster le débit d'écoulement réel du gaz anesthésique résiduel conformément à la commande de contrôle de débit d'écoulement.

6. Le système de distribution de gaz anesthésique (10) de la revendication 5, dans lequel la commande de contrôle de débit d'écoulement est un point de consigne de débit d'écoulement, et le système de contrôle de récupérateur (30) fonctionne conformément à un contrôle en boucle fermée moyennant quoi le circuit de contrôle électronique (50) contrôle automatiquement la valve de contrôle (48) afin d'obtenir le point de consigne de débit d'écoulement.

7. Le système de distribution de gaz anesthésique (10) de la revendication 5 ou de la revendication 6, dans lequel la commande de contrôle de débit d'écoulement est une entrée d'ajustement de débit d'écoulement pour augmenter ou diminuer le débit d'écoulement réel, et le système de contrôle de récupérateur (30) fonctionne conformément à un contrôle en boucle ouverte moyennant quoi le circuit de contrôle électronique (50) contrôle la valve de contrôle (48) conformément à l'entrée d'ajustement de débit d'écoulement.

8. Le système de distribution de gaz anesthésique (10) de n'importe lesquelles des revendications 1 à 7, dans lequel l'interface utilisateur (34) inclut un écran d'affichage (36) pour afficher les informations de débit d'écoulement.

9. Le système de distribution de gaz anesthésique (10) de n'importe lesquelles des revendications 1 à 8, comprenant en outre un appareil de respiration en communication fluidique avec la machine à gaz (12) et le système de contrôle de récupérateur (30) ; dans lequel l'appareil de respiration comprend un conduit d'inhalation (22) qui transmet le gaz anesthésique de la machine à gaz (12) au patient, un masque nasal (26) qui est porté par le patient pour inhaler au moins une partie du gaz anesthésique, et un conduit d'évacuation (28) qui transmet le gaz anesthésique résiduel du masque nasal (26) au système de contrôle de récupérateur (30).

10. Le système de distribution de gaz anesthésique (10) de la revendication 9, comprenant en outre un raccord en T pour poche (18) qui est en communication fluidique avec la machine à gaz (12) et l'appareil de respiration (22, 26, 28), et une poche réservoir (20) qui est en communication fluidique avec le raccord en T pour poche (18) le gaz anesthésique s'écoulant à travers le raccord en T pour poche (18) pour remplir la poche réservoir (20).

11. Le système de distribution de gaz anesthésique (10) de n'importe lesquelles des revendications 1 à 10, le système de distribution de gaz anesthésique étant un système de distribution d'oxyde nitreux, la machine à gaz (12) a une première entrée (14) pour recevoir un apport d'oxyde nitreux et une deuxième entrée (16) pour recevoir un apport d'oxygène, et la machine à gaz (12) mélangeant l'oxyde nitreux et l'oxygène afin de former un gaz anesthésique mélangé qui est apporté au patient.

12. Le système de distribution de gaz anesthésique (10) de n'importe quelle revendication précédente, dans lequel le capteur d'écoulement d'air (46) est un capteur thermique.
